# EUROPEAN PATENT APPLICATION

(11) **EP 2 491 812 A2**
(43) Date of publication of application: **29.08.2012**
(21) Application number: 12155071.9
(22) Date of filing: 13.02.2012
(51) Int. Cl.: A45D 29/00, A61B 17/54

(54) **Abrasive stone**

(30) Priority: 23.02.2011 ES 201100176 U
(71) Applicant: Cuadrillero Altava, M. Teresa, 28010 Madrid (ES); Paniagua Garcia, M. Teresa, 28010 Madrid (ES); Pietsch Cuadrillero, M. Teresa, 28010 Madrid (ES); Pietsch Caudrillero, Juan Carlos, 28010 Madrid (ES)
(72) Inventor: Pietsch Cuadrillero, Juan Carlos, 28010 Madrid (ES)
(74) Representative: Isern-Jara, Nuria

(57) **Abstract**

Abrasive stone (1), of the type used in cosmetics, feet care, hand care or other fields, which may adopt any particular shape, size and colour, incorporing a removable handle (2) to permit an easier handling and operation of the stone (1), having coupling means (3,4) capable of mating together in the stone (1) and in the handle (2), with the possibility of both elements being mutually separated apart and reducing the space occupied by the assembly of the stone (1) and the handle (2).

## Description

### Subject of the invention

The invention refers to an abrasive stone, specifically one of the type used in cosmetics, feet care and hand care.

The objective of the invention is to provide a handle to the abrasive stone to make its use and handling easier.

### Background to the invention

As is well known in hand and feet care, as well as in other fields or branches, abrasive stones are used which may adopt any shape, size and colour. The stones are to be grasped with the fingers, which is not very convenient at the moment of carrying out its handling and the corresponding application on the body of the user.

### Description of the invention

The abrasive stone of the invention which, as above stated, may adopt any form, size and colour, has the peculiarity to incorporate a removable handle to permit an easier handling and operation of the stone.

In concrete terms, the removable handle is fixed to the abrasive stone by means of a threaded lug or screw which may have been previously secured at one of the ends of the stone; which lug is to be screwed within a hole which has been made with this objective at the end of the handle. Eventually the lug may protrude from said end for the handle, being screwed in a hollow block secured to the stone.

In this way, a stone with handle is obtained that as above stated, may be easily handled in its use. It is advantageous that the handle may be removed from the stone for its storage, marketing or for any other eventuality in which there may be the need to reduce the space occupied by the assembly of the stone and the handle.

Concerning the characteristics of the handle, these may be variable as to the material which may be wood, metal, plastic or others with the possibility of having a hole or hook for its hanging when not in use.

### Description of the drawings

To complete the description which will be made in the following and to assist to a better understanding of the features of the invention according to a preferred embodiment, a set of drawings is appended to the description, forming part of the same, in which drawings the following has been shown in an illustrative but not limitative way:

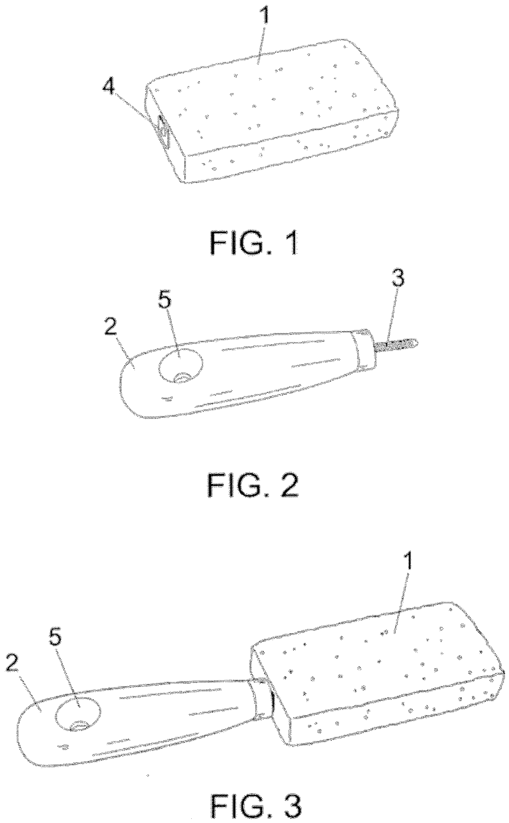

Figure 1.- A general perspective view of an abrasive stone having a prismatic rectangular shape, with a threaded hole to incorporate the handle.
Figure 2.- A perspective view of the handle with a threaded lug to incorporate the abrasive stone shown in the previous figure.
Figure 3.- Aperspective view of the assembly of the abrasive stone and the handle properly coupled together.

### Preferred embodiment of the invention

As may be seen in the appended drawings, the abrasive stone (1) which is the object of the invention, has been shown with a prismatic rectangular shape, being however possible for it to adopt any other configuration, and it has the peculiarity that the stone is designed to be incorporated to a handle (2) to easy up the manipulation and use of the stone to which end the abrasive stone (1) or alternatively the handle (2) are provided with a protruding threaded lug or screw (3), that in the case as shown protrudes from said handle. Said lug or screw (3) is aimed at being incorporated by screwing within a hollow screwed hole (4) provided in this case in the abrasive stone (1) although, as already stated, the threaded lug or screw (3) and the threaded hole (4) may be made either in the handle (2) or in the abrasive stone (1).

Independently of the different shapes which may adopt the abrasive stone (1) as well as different sizes and colours, the same being valid for the handle (2), this latter may have a through opening (5) to permit the hanging of the assembly formed by the coupling of the abrasive stone (1) and the handle (2) as shown in figure (3), thus permitting an easy storage of the abrasive stone when not in use, as it may be hung from a hook or similar.

Likewise, for the storage or keeping of the abrasive stone as well as for its marketing, the abrasive stone (1) and the handle (2) may be dealt independently to occupy a more reduced volume or a minor length as the coupling of both parts is carried out very easily, being sufficient to screw a lug within a threaded hole.

## Claims

1. Abrasive stone, of the type used in feet care, hand care or other fields, which may adopt any particular shape, size and colour **characterised in that** it incorporates a removable handle to facilitate its use, having coupling means capable of mating together in the stone and in the handle, with the possibility of both elements being mutually separated apart.

2. Abrasive stone, according to claim 1, **characterised in that** the means to couple together the abrasive stone and the handle, consist in a threaded lug protruding from one of the ends of the abrasive stone, which lug may be assembled by screwing within a threaded hole of the corresponding end of the handle.

3. Abrasive stone, according to claim 1, **characterised in that** the means to couple together the abrasive stone and the handle consist in a threaded lug protruding from the end of the handle and a block with a hole correspondingly incorporated in the abrasive stone.
